# EUROPÄISCHE PATENTSCHRIFT

(11) **EP 1 587 877 B1**
(45) Veröffentlichungstag und Bekanntmachung des Hinweises auf die Patenterteilung: **14.06.2006**
(21) Anmeldenummer: 03789269.2
(22) Anmeldetag: 13.12.2003
(51) Int. Cl.: C09B 7/00, C09B 57/04, C07D 403/14, C07D 209/44

(54) **BETA-ISOINDIGOFARBMITTEL**
BETA-ISOINDIGO COLORING AGENT
COLORANT BETA-ISOINDIGO

(30) Priorität: 21.01.2003 DE 10302020
(43) Veröffentlichungstag der Anmeldung: 26.10.2005
(73) Patentinhaber: Clariant Produkte (Deutschland) GmbH, 65929 Frankfurt am Main (DE)
(72) Erfinder: HECKMANN, Heino, 65835 Leiderbach (DE); METZ, Hans-Joachim, 64285 Darmstadt (DE)
(86) Internationale Anmeldenummer: PCT/EP2003/014201
(87) Internationale Veröffentlichungsnummer: WO 2004/065490

(56) Entgegenhaltungen:
- EP-A- 0 101 954
- EP-A- 0 190 692
- US-A- 2 254 354

## Beschreibung

Die vorliegende Erfindung betrifft neue organische Pigmente und Farbstoffe.

Auf dem Gebiet der Farbmittel besteht eine ständige Marktnachfrage nach neuen Farbnuancen, die hohe Migrations- und Lichtechtheiten, gute Wärmestabilitäten und eine hohe Färbekraft, sowie im Falle von Pigmenten zusätzlich hohe Lösungsmittelechtheiten aufweisen.

US 2,254,354 beschreibt β-Isoindigoverbindungen, die mit einem cyclischen Rest, der mindestens eine Gruppe =CH₂-CO- enthält, substituiert sind. Die dort genannten Verbindungen sind jedoch trübe.

Es bestand die Aufgabe, verbesserte β-Isoindigopigmente bereitzustellen, die neben den oben genannten pigmentären Eigenschaften eine höhere Reinheit und Brillanz als die im Stand der Technik aufweisen.

Es wurde gefunden, dass diese Aufgabe überraschenderweise durch Verbindungen der Formel (1) gelöst wird.

Gegenstand der vorliegenden Erfindung sind somit Verbindungen der allgemeinen Formel (I) 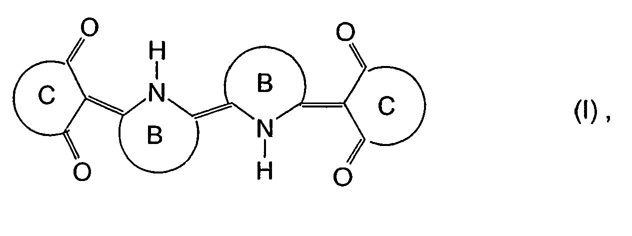 wobei C eine alicyclische oder heterocyclische Gruppe mit C₂ᵥ-Symmetrie
bedeutet,
und B für ortho-C₆-C₁₈-Arylen steht.

Vorzugsweise sind die Verbindungen der Formel (I) symmetrisch, d.h. die Gruppen B sind jeweils gleich, und die Gruppen C sind jeweils gleich.

Bevorzugt sind weiterhin Verbindungen der Formel (I), worin B ortho-Phenylen oder 2,3-Naphthylen ist.

Bevorzugt sind weiterhin Verbindungen der allgemeinen Formel (I) worin der Ring C einem Ringsystem mit C₂ᵥ-Symmetrie der Formeln (a) bis (d) entspricht, 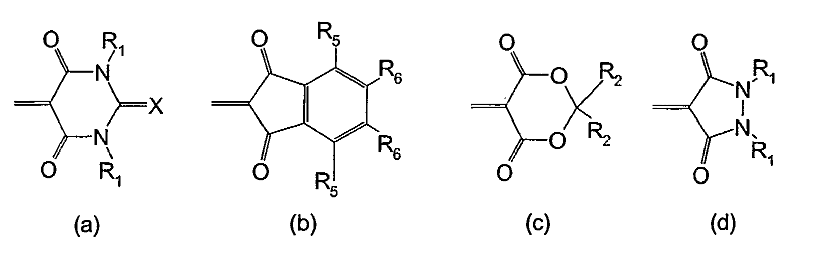 wobei R₁ und R₂ unabhängig voneinander für Wasserstoff, C₁-C₂₅-Alkyl, C₅-C₁₂₋Cycloalkyl, C₆-C₂₄-Aryl, C₁-C₂₅-Alkyl-(C₆-C₁₀)-aryl, einen heteroaromatischen Rest mit 1, 2 oder 3 Heteroatomen aus der Gruppe N, O und S, -(CH₂)ₙ-COR₃ oder -(CH₂)ₘ-OR₄, stehen,
worin R₃ für Hydroxy, unsubstituiertes oder ein- oder mehrfach mit Hydroxy oder Amino substituiertes C₁-C₂₅-Alkoxy, C₁-C₂₅-Alkylamino, Di-(C₁-C₂₅-alkyl)-amino, C₁-C₂₅-Alkyl-C₆-C₂₄-aryl-amino, (C₆-C₂₄-Aryl)-amino, Di-(C₆-C₂₄-Aryl)-amino oder C₂-C₂₄-Alkenyloxy steht,
und R₄ für Wasserstoff oder -CO-(C₁-C₂₅-Alkyl) steht, und n und m unabhängig voneinander für eine ganze Zahl von 0 bis 6, bevorzugt 1 bis 4, stehen, und worin in R₁, R₂, R₃ und R₄ eine C-C-Einheit auch durch eine Ethereinheit C-O-C ersetzt sein kann,
und X für =O, =S oder =NR₂ steht,
und R₅ und R₆ unabhängig voneinander Wasserstoff, Halogen, CN, R₁, OR₁, SR₁, NR₁R₂, NO₂, SO₂(OR₁), SO₂R₁, SO₂NR₁R₂ oder PO₂(OR₁) bedeuten.
Der Begriff der C₂ᵥ-Symmetrie ist in der Fachliteratur beschrieben.

Die Substituenten C in Verbindungen der Formel (I) besitzen als C₂ᵥ-symmetrische Moleküle die Symmetrieelemente der Identität, eine C₂-Achse und zwei orthogonal zueinander stehende Spiegelebenen, deren Schnittgerade die C₂-Achse ergibt.

R₁ und R₂ sind besonders bevorzugt Wasserstoff, C₁-C₁₈-Alkyl, C₅-C₆-Cycloalkyl, C₆-C₁₀-Aryl, Benzyl, Pyridyl, Pyrryl, Thienyl, Imidazolyl, Oxazolyl, Thiazolyl, Pyrimidyl, Hydroxycarbonyl-C₀-C₁₈-alkyl, C₁-C₁₈-Alkoxycarbonyl-C₀-C₁₈-alkyl, Aminocarbonyl-C₀-C₁₈-alkyl, C₁-C₁₈-Alkylaminocarbonyl-C₀-C₁₈-alkyl, C₆-C₁₀₋Arylaminocarbonyl-C₀-C₁₈-alkyl, Di(C₁-C₁₈-alkyl)-aminocarbonyl-C₀-C₁₈-alkyl, C₁-C₁₈-Alkyl-C₆-C₁₀-arylaminocarbonyl-C₀-C₁₈-alkyl und Di(C₆-C₁₀-aryl)-aminocarbonyl-C₀-C₁₈-alkyl.

R₃ ist besonders bevorzugt Hydroxy, C₁-C₁₈-Alkoxy, C₁-C₁₈-Alkylamino, Di(C₁-C₁₈₋alkyl)-amino, Benzylamino, C₆-C₁₀-Arylamino, Di(C₆-C₁₀-aryl)-amino oder (C₂-C₁₈)-Alkenyloxy.

R₅ und R₆ sind besonders bevorzugt Wasserstoff, CI, Br, C₁-C₁₈-Alkyl, C₅-C₆₋Cycloalkyl, Benzyl, C₆-C₁₀-Aryl, Pyridyl, Pyrryl, Thienyl, Imidazolyl, Oxazolyl, Thiazolyl, Pyrimidyl, C₁-C₁₈-Alkoxy, C₆-C₁₀-Aryloxy, C₁-C₁₈-Alkylthio, C₆-C₁₀₋Arylthio, C₁-C₁₈-Alkylamino, C₆-C₁₀-Arylamino, Di(C₁-C₁₈-alkyl)-amino, C₁-C₁₈₋Alkyl-C₆-C₁₀-arylamino, Di(C₆-C₁₀-aryl)-amino, SO₃H, C₁-C₁₈-Alkoxysulfonyl, C₁₋C₁₈-Alkylsulfonyl und Di(C₁-C₁₈-alkyl)-aminosulfonyl.

Von besonderem Interesse sind die Verbindungen der Formel (2) und (3) 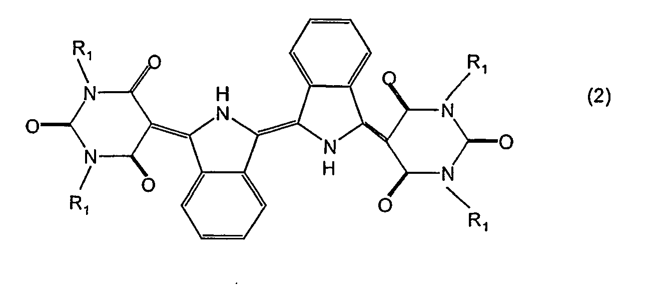 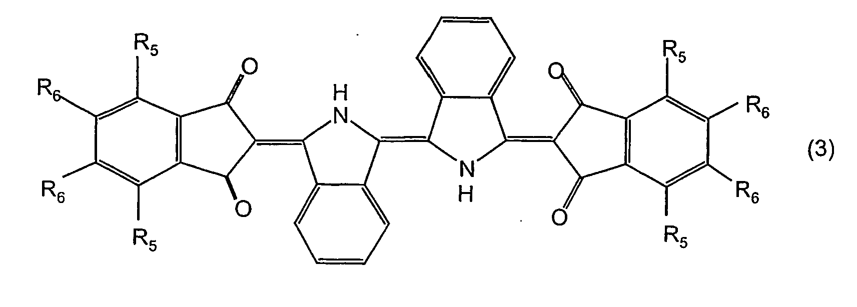 worin R₁, R₅ und R₆ die vorstehenden Bedeutungen haben.

Ein weiterer Gegenstand der vorliegenden Erfindung ist ein Verfahren zur Herstellung von Verbindungen der allgemeinen Formel (I) durch Kondensation einer Verbindung der Formel (III), 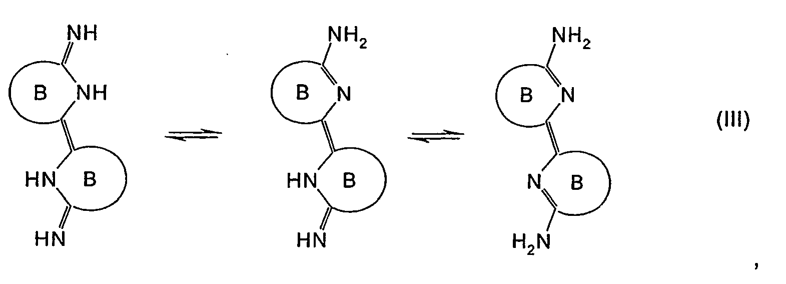 mit mindestens 2 Molequivalenten einer cyclischen Verbindung der Formel (IV),

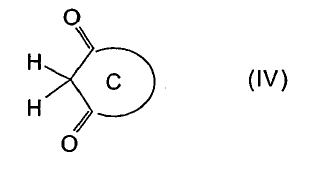

Zweckmäßigerweise erfolgt die Umsetzung bei einer Temperatur von 10 bis 250°C, bevorzugt 100 bis 200°C, vorzugsweise in einem hochsiedenden Lösemittel, wie N-Methyl-pyrrolidon, Propylenglykol, 2-Phenoxyethanol, Chlorbenzol, 1.,2-Dichlorbenzol, 1-Chlornaphthalin, N,N-Dimethylanilin, sowie gegebenenfalls in Gegenwart einer organischen Säure, wie z.B. Ameisensäure, Essigsäure und Propionsäure, oder in Gegenwart einer anorganischen Säure, wie z.B. Schwefelsäure, Salzsäure und Phosphorsäure.

Die Verbindungen der allgemeinen Formel (III) können nach einem an sich bekannten Verfahren (R.P. Smirnov et al, Izv. Vysshikh, Uchebn.Zavednii, Khim. i Khim. Tekhnol. 1963, 6, 1022-4) durch Umsetzung von [1,1']Biisoindolyliden-3,3'-dithionen der Formel (V) mit konzentrierter wässriger Ammoniaklösung im Autoklav in Gegenwart von Natriumnitrit bei 110°C erhalten werden.

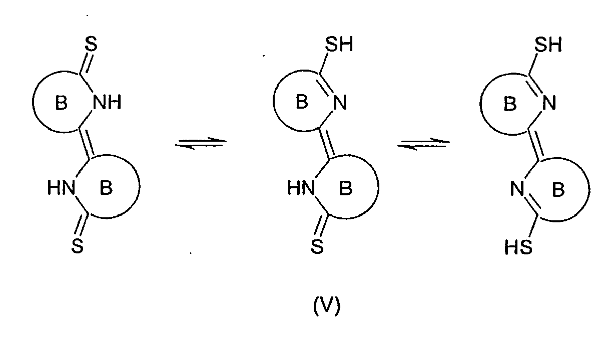

Erfindungsgemäße Verbindungen der allgemeinen Formel (I) werden zum Einfärben oder Pigmentieren von organischen oder anorganischen, hoch- oder niedermolekularen, insbesondere hochmolekularen organischen Materialien verwendet.

Je nach Art ihrer Substituenten und des zu färbenden hochmolekularen organischen Materials können die erfindungsgemäßen Verbindungen als polymerlösliche Farbstoffe oder als Pigmente verwendet werden. Im letzteren Fall ist es vorteilhaft, die bei der Synthese anfallenden Produkte (Rohpigmente) durch Nachbehandlung in organischen Lösungsmitteln, in denen die Pigmente selbst nicht gelöst werden, und bei erhöhten Temperaturen, beispielsweise bei 60 bis 200°C, insbesondere bei 70 bis 150°C, vorzugsweise bei 75 bis 100°C, in eine feindisperse Form mit oft weiter verbesserten Pigmenteigenschaften zu überführen. Die Nachbehandlung wird vorzugsweise mit einer Mahl- oder Knetoperation kombiniert.

Die erfindungsgemäßen Farbmittel eignen sich ausgezeichnet zum Färben von hochmolekularen Materialien, die organischer oder anorganischer Natur sein können, und Kunststoffe und/oder Naturstoffe bedeuten. Es kann sich zum Beispiel um Naturharze, trocknende Öle, Kautschuk oder Casein handeln. Es kann sich aber auch um abgewandelte Naturstoffe handeln, wie beispielsweise Chlorkautschuk, ölmodifizierte Alkydharze, Viskose, Cellulosederivate, wie Celluloseester oder Celluloseether, und insbesondere um vollsynthetische organische Polymere (Kunststoffe), die durch Polymerisation, Polykondensation oder Polyaddition erhalten werden können. Aus der Klasse der durch Polymerisation hergestellten Kunststoffe seien besonders folgende genannt: Polyolefine, wie zum Beispiel Polyethylen, Polypropylen, Polyisobutylen, und substituierte Polyolefine, wie beispielsweise Polystyrol, Polyvinylchlorid, Polyvinylidenchlorid, Polyvinylacetale, Polyacrylnitril, Polyacrylsäure, Polymethacrylsäure, Polyacrylsäure- und Polymethacrylsäureester oder Polybutadien, sowie Copolymerisate davon.
Aus der Klasse der durch Polyaddition und Polykondensation hergestellten Kunststoffe seien genannt: Polyester, Polyamide, Polyimide, Polycarbonate, Polyurethane, Polyether, Polyacetale, sowie die Kondensationsprodukte von Formaldehyd mit Phenolen (Phenoplaste) und die Kondensationsprodukte von Formaldehyd mit Harnstoff, Thioharnstoff und Melamin (Aminoplaste). Weiterhin kann es sich auch um Silikone oder Silikonharze handeln.

Solche hochmolekularen Materialien können einzeln oder in Gemischen als plastische Massen, Schmelzen oder in Form von Spinnlösungen vorliegen. Sie können auch in Form ihrer Monomeren oder im polymerisierten Zustand in gelöster Form als Filmbildner oder Bindemittel für Lacke oder Druckfarben vorliegen, wie Leinölfirnis, Nitrocellulose, Alkydharze, Melaminharze und Formaldehydharze oder Acrylharze.

Die erfindungsgemäßen Verbindungen eignen sich demzufolge als Farbmittel in Anstrichfarben auf öliger oder wässriger Grundlage, in Lacken verschiedener Art, Tarnfarben, zum Spinnfärben, zum Massefärben oder Pigmentieren von Kunststoffen, in Druckfarben für das graphische Gewerbe, wie zum Beispiel im Papier-, Textil- oder Dekorationsdruck, und in der Papiermassefärbung, zur Herstellung von Tinten, Ink-Jet Tinten auf wässriger oder nichtwässriger Basis, Mikroemulsionstinten und Tinten, die nach dem Hot-melt Verfahren arbeiten.

Die erfindungsgemäßen Verbindungen sind auch geeignet als Farbmittel in elektrophotographischen Tonern und Entwicklern, wie beispielsweise Ein- oder Zweikomponentenpulvertonern (auch Ein- oder Zweikomponenten-Entwickler genannt), Magnettoner, Flüssigtoner, Polymerisationstoner sowie Spezialtoner. Typische Tonerbindemittel sind Polymerisations-, Polyadditions- und Polykondensationsharze, wie Styrol-, Styrolacrylat-, Styrolbutadien-, Acrylat-, Polyester-, Phenol-Epoxidharze, Polysulfone, Polyurethane, einzeln oder in Kombination, sowie Polyethylen und Polypropylen, die noch weitere Inhaltsstoffe, wie Ladungssteuermittel, Wachse oder Fließhilfsmittel, enthalten können oder im Nachhinein mit diesen Zusätzen modifiziert werden.

Des weiteren sind die erfindungsgemäßen Verbindungen geeignet als Farbmittel in Pulver und Pulverlacken, insbesondere in triboelektrisch oder elektrokinetisch versprühbaren Pulverlacken, die zur Oberflächenbeschichtung von Gegenständen aus beispielsweise Metall, Holz, Kunststoff, Glas, Keramik, Beton, Textilmaterial, Papier oder Kautschuk zur Anwendung kommen. Als Pulverlackharze werden typischerweise Epoxidharze, carboxyl- und hydroxylgruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harzsystem) sind beispielsweise Säureanhydride, Imidazole sowie Dicyandiamid und deren Abkömmlinge, verkappte Isocyanate, Bisacylurethane, Phenol- und Melaminharze, Triglycidylisocyanurate, Oxazoline und Dicarbonsäuren.

Außerdem sind die erfindungsgemäßen Verbindungen als Farbmittel in Ink-Jet Tinten auf wässriger und nichtwässriger Basis sowie in solchen Tinten, die nach dem hot-melt-Verfahren arbeiten, geeignet.

Ink-Jet-Tinten enthalten im allgemeinen insgesamt 0,5 bis 15 Gew.-%, vorzugsweise 1,5 bis 8 Gew.-%, (trocken gerechnet) einer oder mehrerer der erfindungsgemäßen Verbindungen.

Mikroemulsionstinten basieren auf organischen Lösemitteln, Wasser und ggf. einer zusätzlichen hydrotropen Substanz (Grenzflächenvermittler). Mikroemulsionstinten enthalten im allgemeinen 0,5 bis 15 Gew.-%, vorzugsweise 1,5 bis 8 Gew.-%, einer oder mehrerer der erfindungsgemäßen Verbindungen, 5 bis 99 Gew.-% Wasser und 0,5 bis 94,5 Gew.-% organisches Lösungsmittel und/oder hydrotrope Verbindung.
"Solvent based" Ink-Jet-Tinten enthalten vorzugsweise 0,5 bis 15 Gew.-% einer oder mehrerer der erfindungsgemäßen Verbindungen, 85 bis 99,5 Gew.-% organisches Lösungsmittel und/oder hydrotrope Verbindungen.
Hot-Melt-Tinten basieren meist auf Wachsen, Fettsäuren, Fettalkoholen oder Sulfonamiden, die bei Raumtemperatur fest sind und bei Erwärmen flüssig werden, wobei der bevorzugte Schmelzbereich zwischen ca. 60°C und ca. 140°C liegt. Hot-Melt Ink-Jet-Tinten bestehen z.B. im wesentlichen aus 20 bis 90 Gew.-% Wachs und 1 bis 10 Gew.-% einer oder mehrerer der erfindungsgemäßen Verbindungen. Weiterhin können 0 bis 20 Gew.-% eines zusätzlichen Polymers (als "Farbstofflöser"), 0 bis 5 Gew.-% Dispergierhilfsmittel, 0 bis 20 Gew.-% Viskositätsveränderer, 0 bis 20 Gew.-% Plastifizierer, 0 bis 10 Gew.-% Klebrigkeitszusatz, 0 bis 10 Gew.-% Transparenzstabilisator (verhindert z.B. Kristallisation der Wachse) sowie 0 bis 2 Gew.-% Antioxidans enthalten sein.

Weiterhin sind die erfindungsgemäßen Farbmittel auch für Farbfilter, sowohl für die additive wie auch für die subtraktive Farberzeugung, sowie als Farbmittel für elektronische Tinten ("electronic inks" bzw. "e-inks") oder "electronic paper" ("e-paper") geeignet.

Bei der Herstellung sogenannter Farbfilter, sowohl reflektierender wie durchsichtiger Farbfilter, werden Pigmente in Form einer Paste oder als pigmentierte Photoresists in geeigneten Bindemitteln (Acrylate, Acrylester, Polyimide, Polyvinylalkohole, Epoxide, Polyester, Melamine, Gelantine, Caseine) auf die jeweiligen LCD-Bauteilen (z. B. TFT-LCD= Thin Film Transistor Liquid Crystal Displays oder z.B. ((S) TN-LCD = (Super) Twisted Nematic-LCD) aufgebracht. Neben einer hohen Thermostabilität ist für eine stabile Paste bzw. einem pigmentierten Photoresist auch eine hohe Pigmentreinheit Voraussetzung.

Darüber hinaus können die pigmentierten Color Filter auch durch Ink Jet-Druckverfahren oder andere geeignete Druckverfahren aufgebracht werden.

Die vorliegende Erfindung betrifft überdies die Verwendung der erfindungsgemäßen Farbmittel in optischen Schichten für die optische Datenspeicherung, bevorzugt für die optische Datenspeicherung, bei der ein Laser zum Schreiben der Daten verwendet wird. Die für diese Anwendung notwendige Löslichkeit der Farbmittel im Anwendungsmedium kann durch die Art und Anzahl der Substituenten eingestellt werden.

Des weiteren eignen sich die erfindungsgemäßen Verbindungen als Farbmittel in Kosmetik, zur Einfärbung von Saatgut und zur Einfärbung von Mineralölen, Schmierfetten und Wachsen.

Je nach Art der Substituenten der erfindungsgemäßen Verbindungen, zeichnen sich die erhaltenen Färbungen durch gute Hitze-, Licht- und Wetterechtheit, Chemikalienbeständigkeit und die sehr guten applikatorischen Eigenschaften, z.B. Kristallisierechtheit und Dispergierechtheit und insbesondere durch ihre Migrier-, Ausblüh-, Überlackier- und Lösungsmittelechtheit aus. Die als polymerlösliche Farbstoffe eingesetzten Verbindungen weisen naturgemäß nur eine geringe oder eingeschränkte Lösungsmittelechtheit auf.
Einen weiteren Erfindungsgegenstand bildet eine Zusammensetzung, enthaltend ein organisches oder anorganisches, hochmolekulares oder niedermolekulares, insbesondere hochmolekulares organisches Material, und mindestens eine erfindungsgemäße Verbindung in einer färberisch wirksamen Menge, in der Regel im Bereich von 0,005 bis 70 Gew.-%, insbesondere von 0,01 bis 10 Gew.-%, bezogen auf das organische oder anorganische Material.

### Beispiel 1: 3,3'-Bis-(1,3-diethyl-2-thio-4,6-dioxo-tetrahydropyrimidin-5-yliden)-[1,1']biisoindolyliden

10 g [1,1']Biisoindolyliden-3,3'-diimin und 23,1 g 1,3-Diethyl-2-thiobarbitursäure werden in einer Mischung aus 100 ml NMP und 100 ml Eisessig 4 Stunden bei 155°C gerührt. Die Suspension wird nach Abkühlen auf Raumtemperatur filtriert, mit Ethanol, dann Wasser gewaschen und bei 60°C getrocknet. Es werden 21,3 g (88 %) eines metallisch grünen Pulvers einer Verbindung folgender Formel erhalten 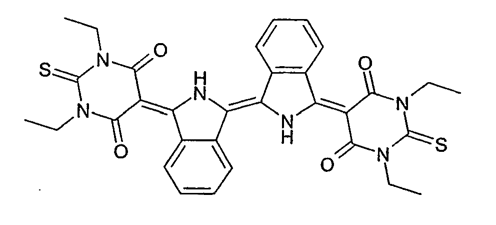 Schmelzpunkt: >300°C
H-NMR (D₂SO₄): 8.05 (d, 2H), 7.80 (d, 2H), 7.54 (t, 2H), 7.33 (t, 2H), 3.95 (s, breit, 8H), 0.95 (t, 12H).

### Beispiel 2: 3,3'-Bis-(1,3-dimethyl-2,4,6-trioxo-tetrahydropyrimidin-5-yliden)-[1,1']büsoindolyliden

10 g [1,1']Biisoindolyliden-3,3'-diimin und 18,1 g 1,3-Dimethylbarbitursäure werden in einer Mischung aus 160 ml NMP und 40 ml Eisessig 5 Stunden am Rückfluss gerührt. Die Suspension wird nach Abkühlen auf Raumtemperatur filtriert, mit Ethanol, dann Wasser gewaschen und bei 60°C getrocknet. Es werden 14,5 g (70 %) eines nahezu schwarzen Pulvers einer Verbindung folgender Formel erhalten 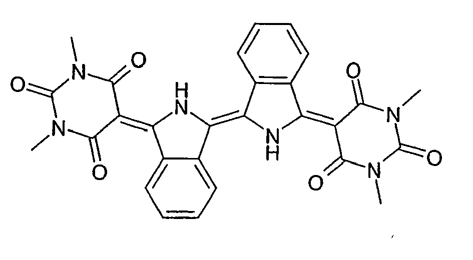 Schmelzpunkt: > 300°C
MS (m/e): 539 [M+H]⁺, 561 [M+Na]⁺

### Beispiel 3: 3,3'-Bis-(1,3-bis-(2-ethylhexyl)-2,4,6-trioxo-tetrahydropyrimidin-5-yliden)-[1,1']büsoindolyliden

10 g [1,1']Biisoindolyliden-3,3'-diimin und 40,9 g 1,3-Bis-(2-ethylhexyl)-barbitursäure werden in einer Mischung aus 100 ml NMP und 100 ml Eisessig 4 Stunden bei 155°C gerührt. Die Suspension wird nach Abkühlen auf Raumtemperatur filtriert, mit Ethanol, dann Wasser gewaschen und bei 60°C getrocknet. Es werden 28,8 g (80 %) eines violetten Pulvers einer Verbindung folgender Formel erhalten 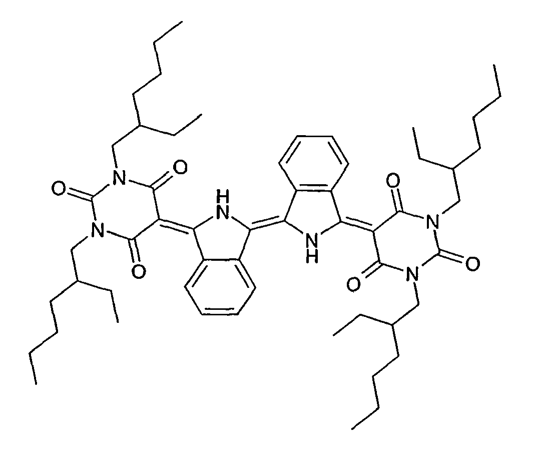 Schmelzbereich: 198-206°C
MALDI (m/e, Negativmodus): 930 [M-H]⁻
H-NMR (CDCl₃): 15.14 (s, 2H), 9.62 (d, 2H), 8.43 (d, 2H), 7.88 (t, 2H), 7.71 (t, 2H), 4.04 (m, 8H), 1.94 (m, 4H), 1.35 (m, 32H), 1.14 (m, 24H)

### Beispiel 4: 3,3'-Bis-(1,3-diphenyl-2,4,6-trioxo-tetrahydropyrimidin-5-yliden)-[1,1']biisoindolyliden

19,4 g [1,1']Biisoindolyliden-3,3'-diimin und 50,0 g 1,3-Diphenylbarbitursäure werden in einer Mischung aus 230 ml NMP und 350 ml Eisessig 6 Stunden am Rückfluss gerührt. Die Suspension wird nach Abkühlen auf Raumtemperatur filtriert, mit Ethanol, dann Wasser gewaschen und bei 60°C getrocknet. Es werden 48,1 g (82 %) eines violetten Pulvers einer Verbindung folgender Formel erhalten 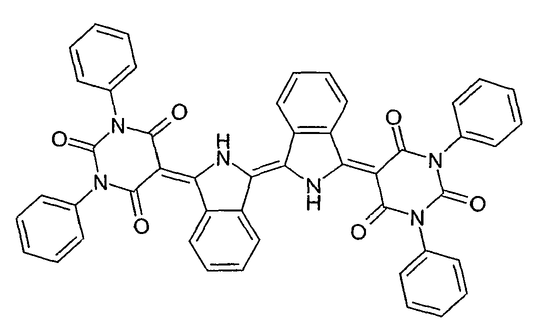 Schmelzpunkt: >300°C
MS (m/e): 787 [M+H]⁺, 809 [M+Na]⁺
H-NMR (D₂SO₄): 7.63 (d, 2H), 7.27 (d, 2H), 7.14 (t, 2H), 6.97 (t, 2H), 6.82 (m, 12H), 6.65 (d, 8H).

### Beispiel 5: 3,3'-Bis-(1,3-dioxoindan-2-yliden)-[1,1']biisoindolyliden

10,0 g [1,1']BÜsoindolyliden-3,3'-dümin und 16,9 g 1,3-Dioxoindan werden in einer Mischung aus 100 ml NMP und 100 ml Eisessig 6 Stunden am Rückfluss gerührt. Die Suspension wird nach Abkühlen auf Raumtemperatur filtriert, mit einem Gemisch aus NMP und Eisessig (1:1), anschließend Ethanol, dann Wasser gewaschen und bei 60°C getrocknet. Es werden 10,8 g (54 %) eines schwarzen Pulvers einer Verbindung folgender Formel erhalten 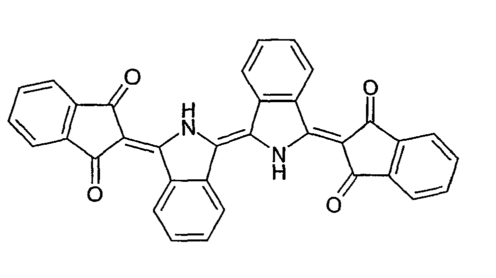 Schmelzpunkt: >300°C
MS (m/e): 519 [M+H]⁺

### Vergleichsbeispiel 1: 3,3'-Bis-(2,4-dioxo-1,4-dihydro-2H-chinolin-3-yliden)-[1,1']büsoindolyliden

5,0 g [1,1']Biisoindolyliden-3,3'-diimin und 14,9 g 1H-Chinolin-2,4-dion werden in einer Mischung aus 90 ml NMP und 10 ml Eisessig 6 Stunden am Rückfluss gerührt. Die Suspension wird nach Abkühlen auf Raumtemperatur filtriert, mit Ethanol, dann Wasser gewaschen und bei 60°C getrocknet. Es werden 6,2 g (59 %) eines nahezu schwarzen Pulvers einer Verbindung folgender Formel erhalten 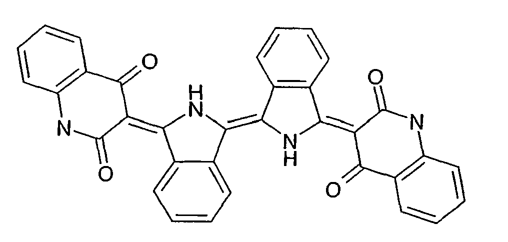 Schmelzpunkt: >300°C
MALDI (m/e): 547 [M-H]⁻

### Vergleichsbeispiel 2: 3,3'-Bis-(3-methyl-5-oxo-1-(3-carboxyphenyl)-1,5-dihydropyrazol-4-yliden)-[1,1']büsoindolyliden

10,0 g [1,1']Biisoindolyliden-3,3'-diimin und 25,3 g 3-Methyl-1-(3-carboxyphenyl)-2-pyrazolin-5-on werden in einer Mischung aus 160 ml NMP und 40 ml Eisessig 5 Stunden am Rückfluss gerührt. Die Suspension wird nach Abkühlen auf Raumtemperatur filtriert, mit Ethanol, dann Wasser gewaschen und bei 60°C getrocknet. Es werden 21,7 g (85 %) eines bräunlich schwarzen Pulvers einer Verbindung folgender Formel erhalten 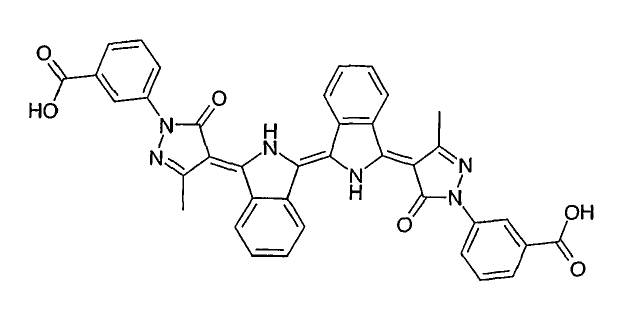 Schmelzpunkt: >300°C
MS (m/e): 661 [M-H]⁻

### Vergleichsbeispiel 3: 3,3'-Bis-(3-methyl-5-oxo-1-(3-sulfamoylphenyl)-1,5-dihydropyrazol-4-yliden)-[1,1']biisoindolyliden

10,0 g [1,1']Biisoindolyliden-3,3'-diimin und 29,4 g 3-Methyl-1-(3-sulfamoyl)-2-pyrazolin-5-on werden in einer Mischung aus 240 ml NMP und 60 ml Eisessig 3 Stunden am Rückfluss gerührt. Die Suspension wird nach Abkühlen auf Raumtemperatur filtriert, mit Ethanol, dann Wasser gewaschen und bei 60°C getrocknet. Es werden 26,7 g (95 %) eines bräunlich schwarzen Pulvers einer Verbindung folgender Formel erhalten 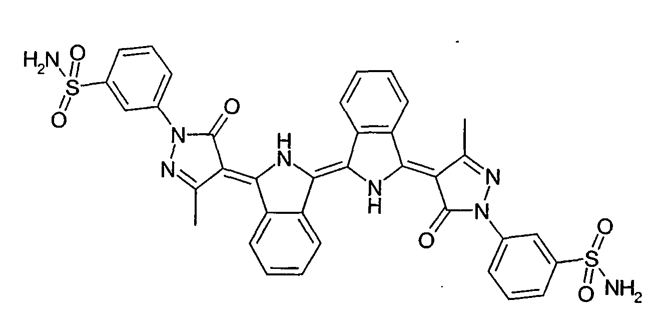 Schmelzpunkt: >300°C
MALDI (m/e): 731 [M-H]⁻

### Anwendungsbeispiele

Zur Beurteilung der Eigenschaften der nach der Erfindung hergestellten Pigmente auf dem Lacksektor wurden aus der Vielzahl der bekannten Lacke ein aromatenhaltiger Alkydmelaminharzlack (AM) auf Basis eines mittelöligen Alkydharzes und eines butanolveretherten Melaminharzes, sowie ein aromatenfreier lufttrocknender Alkydharzlack (LA) auf Basis eines langöligen Sojaalkydharzes ausgewählt.

Zur Beurteilung der Eigenschaften der erfindungsgemäßen polymerlöslichen Farbstoffe wurden glasklares Polystyrol, Polycarbonat oder Polyester als einzufärbende Kunststoffe ausgewählt. Die Herstellung von Prüfkörpern erfolgte durch Spritzgießen.

### Anwendungsbeispiel 1:

Eine Applikation des Pigments aus Beispiel 1 in LA-Lack ergibt farbstarke, im Vollton deckend schwarze, in der Aufhellung rotstichig blaue Lackierungen.

### Anwendungsbeispiel 2:

Eine Applikation des Pigments aus Beispiel 2 in LA-Lack ergibt farbstarke, im Vollton deckend schwarze, in der Aufhellung rotstichig violette Lackierungen.

### Anwendungsbeispiel 3:

Eine Applikation des Pigments aus Beispiel 3 in Polystyrol ergibt im Vollton und in der Aufhellung reine und farbstarke violette Prüfkörper.

### Anwendungsbeispiel 4:

Eine Applikation des Pigments aus Beispiel 3 in Polyethylenterephthalat ergibt im Vollton und in der Aufhellung reine und farbstarke violette Prüfkörper.

### Anwendungsbeispiel 5:

Eine Applikation des Pigments aus Beispiel 4 in AM-Lack ergibt reine und farbstarke, im Vollton dunkle violette, in der Aufhellung rotstichig violette Lackierungen.

### Anwendungsbeispiel 6:

Eine Applikation des Pigments aus Beispiel 4 in Polycarbonat ergibt im Vollton und in der Aufhellung farbstarke, rotstichig violette Prüfkörper, mit roter Fluoreszenz im Vollton.

### Anwendungsvergleichsbeispiel 1:

Eine Applikation des Pigments aus Vergleichsbeispiel 1 in AM-Lack ergibt im Vollton deckend schwarze, in der Aufhellung blaustichig violette Lackierungen, die bedeutend trüber sind als die mit Verbindungen der Beispiele 1 bis 5.

### Anwendungsvergleichsbeispiel 2:

Eine Applikation des Pigments aus Vergleichsbeispiel 2 in AM-Lack ergibt im Vollton deckend schwarze, in der Aufhellung violette Lackierungen, die bedeutend trüber sind als die mit Verbindungen der Beispiele 1 bis 5.

### Anwendungsvergleichsbeispiel 3:

Eine Applikation des Pigments aus Vergleichsbeispiel 2 in AM-Lack ergibt farbstarke, im Vollton deckend schwarze, in der Aufhellung blaue Lackierungen, die bedeutend trüber sind als die mit Verbindungen der Beispiele 1 bis 5.

## Patentansprüche

1. Verbindung der allgemeinen Formel (I) 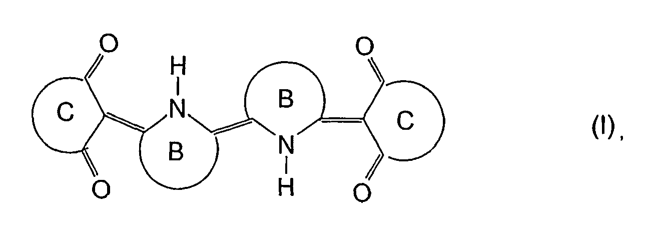 wobei C eine alicyclische oder heterocyclische Gruppe mit C₂ᵥ-Symmetrie
bedeutet,
und B für ortho-C₆-C₁₈-Arylen steht.

2. Verbindung nach Anspruch 1, **dadurch gekennzeichnet, dass** B ortho-Phenylen oder 2,3-Naphthylen ist.

3. Verbindung nach Anspruch 1 oder 2, **dadurch gekennzeichnet, dass** der Ring C einem Ringsystem der Formeln (a) bis (d) entspricht, 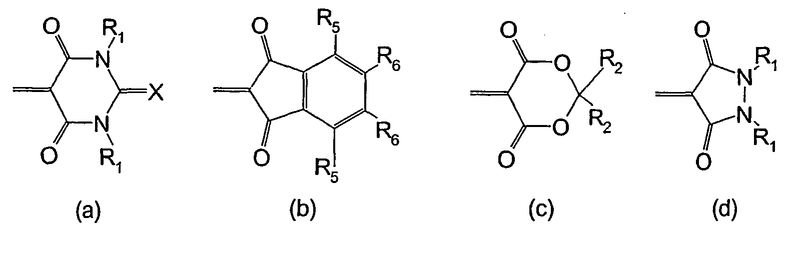 wobei R₁ und R₂ unabhängig voneinander für Wasserstoff, C₁-C₂₅-Alkyl, C₅-C₁₂₋Cycloalkyl, C₆-C₂₄-Aryl, C₁-C₂₅-Alkyl-(C₆-C₁₀)-aryl, einen heteroaromatischen Rest mit 1, 2 oder 3 Heteroatomen aus der Gruppe N, O und S, -(CH₂)ₙ-COR₃ oder -(CH₂)ₘ-OR₄, stehen,
worin R₃ für Hydroxy, unsubstituiertes oder ein- oder mehrfach mit Hydroxy oder Amino substituiertes C₁-C₂₅-Alkoxy, C₁-C₂₅-Alkylamino, Di-(C₁-C₂₅-alkyl)-amino, C₁-C₂₅-Alkyl-C₆-C₂₄-aryl-amino, (C₆-C₂₄-Aryl)-amino, Di-(C₆-C₂₄-Aryl)-amino oder C₂-C₂₄-Alkenyloxy steht,
und R₄ für Wasserstoff oder -CO-(C₁-C₂₅-Alkyl) steht, und n und m unabhängig voneinander für eine ganze Zahl von 0 bis 6, bevorzugt 1 bis 4, stehen, und worin in R₁, R₂, R₃ und R₄ eine C-C-Einheit auch durch eine Ethereinheit C-O-C ersetzt sein kann,
und X für =O, =S oder =NR₂ steht,
und R₅ und R₆ unabhängig voneinander Wasserstoff, Halogen, CN, R₁, OR₁, SR₁, NR₁R₂, NO₂, SO₂(OR₁), SO₂R₁, SO₂NR₁R₂ oder PO₂(OR₁) bedeuten.

4. Verbindung nach einem oder mehreren der Ansprüche 1 bis 3, **dadurch gekennzeichnet, dass** R₁ und R₂ gleich oder verschieden sind und Wasserstoff, C₁-C₁₈-Alkyl, Benzyl, C₅-C₆-Cycloalkyl, C₆-C₁₀-Aryl, Pyridyl, Pyrryl, Thienyl, Imidazolyl, Oxazolyl, Thiazolyl, Pyrimidyl, Hydroxycarbonyl-C₀-C₁₈-alkyl, C₁-C₁₈₋Alkoxycarbonyl-C₀-C₁₈-alkyl, Aminocarbonyl-C₀-C₁₈-alkyl, C₁-C₁₈₋Alkylaminocarbonyl-C₀-C₁₈-alkyl, C₆-C₁₀-Arylaminocarbonyl-C₀-C₁₈-alkyl, Di(C₁₋C₁₈-alkyl)-aminocarbonyl-C₀-C₁₈-alkyl, C₁-C₁₈-Alkyl-C₆-C₁₀-arylaminocarbonyl-C₀₋C₁₈-alkyl oder Di(C₆-C₁₀-aryl)-aminocarbonyl-C₀-C₁₈-alkyl bedeuten.

5. Verbindung nach einem oder mehreren der Ansprüche 1 bis 4, **dadurch gekennzeichnet, dass** R₅ und R₆ gleich oder verschieden sind und Wasserstoff, Cl, Br, C₁-C₁₈-Alkyl, C₅-C₆-Cycloalkyl, C₆-C₁₀-Aryl, Benzyl, Pyridyl, Pyrryl, Thienyl, Imidazolyl, Oxazolyl, Thiazolyl, Pyrimidyl, C₁-C₁₈-Alkoxy, C₆-C₁₀-Aryloxy, C₁-C₁₈₋Alkylthio, C₆-C₁₀-Arylthio, C₁-C₁₈-Alkylamino, C₆-C₁₀-Arylamino, Di(C₁-C₁₈-alkyl)-amino, C₁-C₁₈-Alkyl-C₆-C₁₀-arylamino, Di(C₆-C₁₀-)arylamino, SO₃H, C₁-C₁₈₋Alkoxysulfonyl, C₁-C₁₈-Alkylsulfonyl oder Di(C₁-C₁₈-alkyl)-aminosulfonyl bedeuten.

6. Verbindung der Formel (2) oder (3) gemäß Anspruch 3, 4 oder 5

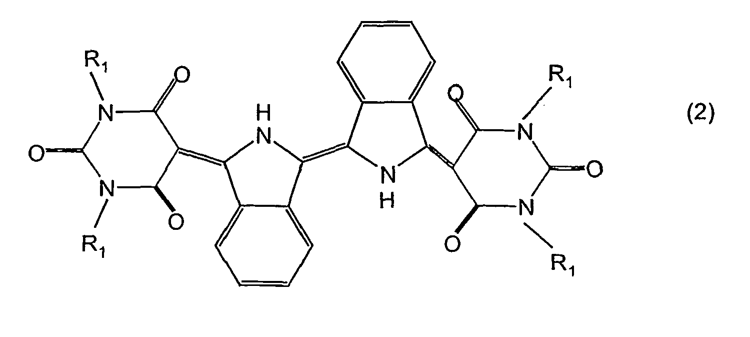

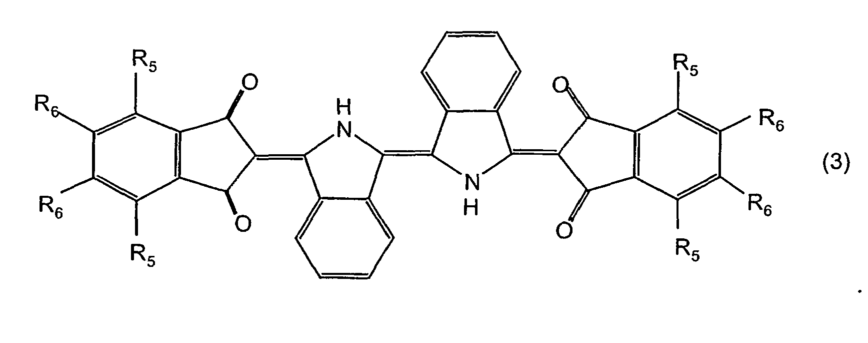

7. Verfahren zur Herstellung einer Verbindung gemäß einem oder mehreren der Ansprüche 1 bis 6, **dadurch gekennzeichnet, dass** man eine Verbindung der Formel (III), 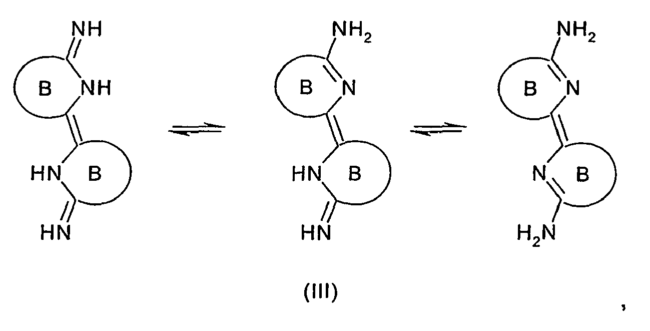 mit mindestens 2 Molequivalenten einer cyclischen Verbindung der Formel (IV) kondensiert

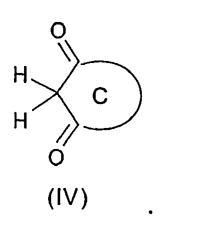

8. Verwendung einer Verbindung gemäß einem oder mehreren der Ansprüche 1 bis 6 zum Einfärben oder Pigmentieren von organischen oder anorganischen, hoch- oder niedermolekularen, insbesondere hochmolekularen organischen Materialien.

9. Verwendung nach Anspruch 8 als Farbmittel in Anstrichfarben auf öliger oder wässriger Grundlage, in Lacken, Tarnfarben, zum Spinnfärben, zum Massefärben oder Pigmentieren von Kunststoffen, in Druckfarben, in der Papiermassefärbung, für Saatgut, Pulverlacke, zur Herstellung von Tinten, Ink-Jet Tinten auf wässriger oder nichtwässriger Basis, Mikroemulsionstinten und Tinten, die nach dem Hot-melt Verfahren arbeiten.

10. Verwendung nach Anspruch 8 als Farbmittel für elektrophotographische Toner und Entwickler, für Farbfilter, für elektronische Tinten, sowie in optischen Schichten für die optische Datenspeicherung.

11. Zusammensetzung, enthaltend ein organisches oder anorganisches, hochmolekulares oder niedermolekulares Material und eine Verbindung nach einem oder mehreren der Ansprüche 1 bis 6 in einer Menge von 0,005 bis 70 Gew.-%, bezogen auf das organische oder anorganische Material.

## Claims

1. A compound of the formula (I) 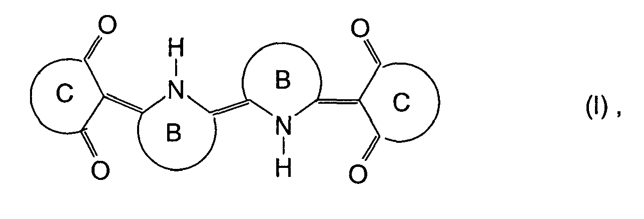 where C is an alicyclic or heterocyclic group having C₂ᵥ symmetry
and B is ortho-C₆-C₁₈ arylene.

2. A compound as claimed in claim 1, wherein B is ortho-phenylene or 2,3-naphthylene.

3. A compound as claimed in claim 1 or 2, wherein the ring C corresponds to a ring system of the formulae (a) to (d) 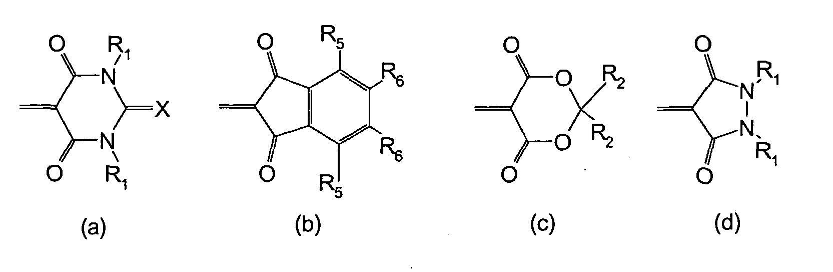 where R₁ and R₂ independently of one another are hydrogen, C₁-C₂₅ alkyl, C₅-C₁₂- cycloalkyl, C₆-C₂₄ aryl, C₁-C₂₅ alkyl- (C₆-C₁₀) -aryl, a heteroaromatic radical having 1, 2 or 3 heteroatoms from the group N, O and S, -(CH₂)ₙ-COR₃ or -(CH₂)ₘ-OR₄,
in which R₃ is hydroxyl, unsubstituted or mono- or poly-hydroxyl- or amino-substituted C₁-C₂₅ alkoxy, C₁-C₂₅ alkylamino, di-(C₁-C₂₅ alkyl) amino, C₁-C₂₅ alkyl-C₆-C₂₄ arylamino, (C₆-C₂₄ aryl) amino, di-(C₆-C₂₄ aryl) amino or C₂-C₂₄ alkenyloxy,
and R₄ is hydrogen or -CO-(C₁-C₂₅ alkyl), and n and m independently of one another are an integer from 0 to 6, preferably 1 to 4, and in which in R₁, R₂, R₃ and R₄ a C-C unit may also be replaced by an ether unit C-O-C, and
X is =O, =S or =NR₂,
and R₅ and R₆ independently of one another are hydrogen, halogen, CN, R₁, OR₁, SR₁, NR₁R₂, NO₂, SO₂(OR₁), SO₂R₁, SO₂NR₁R₂ or PO₂(OR₁).

4. A compound as claimed in one or more of claims 1 to 3, wherein R₁ and R₂ are identical or different and are hydrogen, C₁-C₁₈ alkyl, benzyl, C₅-C₆ cycloalkyl, C₆-C₁₀ aryl, pyridyl, pyrryl, thienyl, imidazolyl, oxazolyl, thiazolyl, pyrimidyl, hydroxycarbonyl-C₀-C₁₈ alkyl, C₁-C₁₈ alkoxycarbonyl-C₀-C₁₈ alkyl, aminocarbonyl-C₀-C₁₈ alkyl, C₁-C₁₈ alkylaminocarbonyl-C₀-C₁₈ alkyl, C₆-C₁₀ arylaminocarbonyl-C₀-C₁₈ alkyl, di(C₁-C₁₈ alkyl) - aminocarbonyl-C₀-C₁₈ alkyl, C₁-C₁₈ alkyl-C₆-C₁₀₋arylaminocarbonyl-C₀-C₁₈ alkyl or di(C₆-C₁₀-aryl)-aminocarbonyl-C₀-C₁₈ alkyl.

5. A compound as claimed in one or more of claims 1 to 4, wherein R₅ and R₆ are identical or different and are hydrogen, Cl, Br, C₁-C₁₈ alkyl, C₅-C₆ cycloalkyl, C₆-C₁₀ aryl, benzyl, pyridyl, pyrryl, thienyl, imidazolyl, oxazolyl, thiazolyl, pyrimidyl, C₁-C₁₈ alkoxy, C₆-C₁₀ aryloxy, C₁-C₁₈ alkylthio, C₆-C₁₀ arylthio, C₁-C₁₈ alkylamino, C₆-C₁₀ arylamino, di(C₁-C₁₈ alkyl)-amino, C₁-C₁₈ alkyl-C₆-C₁₀-arylamino, di(C₆-C₁₀-)arylamino, SO₃H, C₁-C₁₈ alkoxysulfonyl, C₁-C₁₈ alkylsulfonyl or di(C₁-C₁₈ alkyl)-aminosulfonyl.

6. A compound of the formula (2) or (3) as claimed in claim 3, 4 or 5

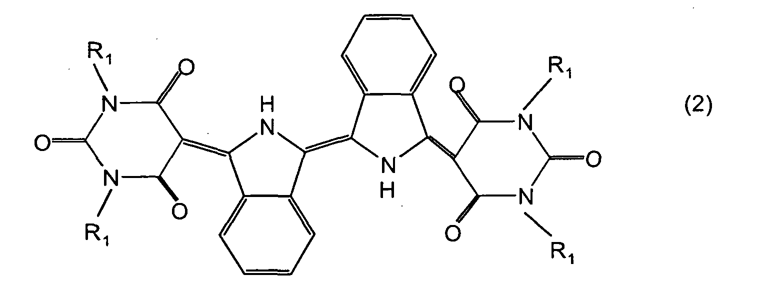

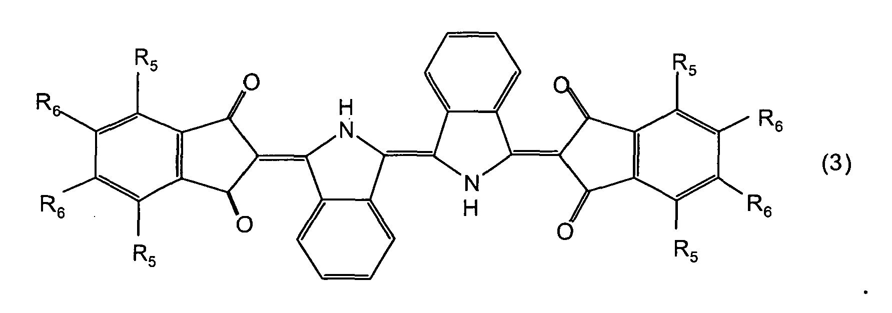

7. A process for preparing a compound as claimed in one or more of claims 1 to 6, which comprises condensing a compound of the formula (III) 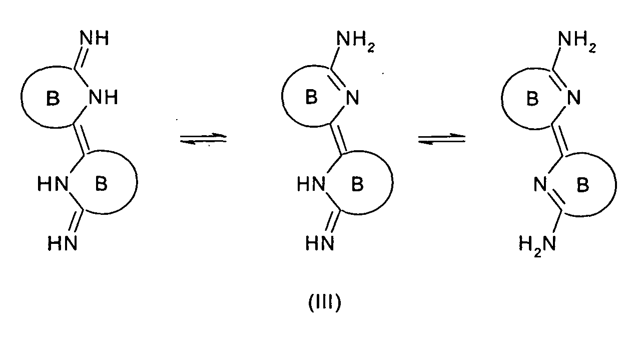 with at least 2 mole equivalents of a cyclic compound of the formula (IV)

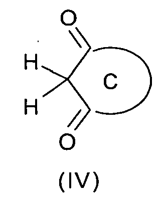

8. The use of a compound as claimed in one or more of claims 1 to 6 for coloring or pigmenting organic or inorganic materials of high or low molecular mass, particularly organic materials of high molecular mass.

9. The use as claimed in claim 8 as a colorant in oil-based or water-based paints, in varnishes, camouflage paints, for spin coloring, for the mass coloring or pigmenting of plastics, in printing inks, in the mass coloring of paper, for seed, powder coating materials, for preparing inks, water-based or non-water-based ink-jet inks, microemulsion inks, and inks which operate in accordance with the hot-melt process.

10. The use as claimed in claim 8 as colorants for electrophotographic toners and developers, for color filters, for electronic inks, and in optical layers for optical data storage.

11. A composition comprising an organic or inorganic material of high or low molecular mass and a compound as claimed in one or more of claims 1 to 6 in an amount of 0.005 to 70% by weight, based on the organic or inorganic material.

## Revendications

1. Composés de formule générale (I) 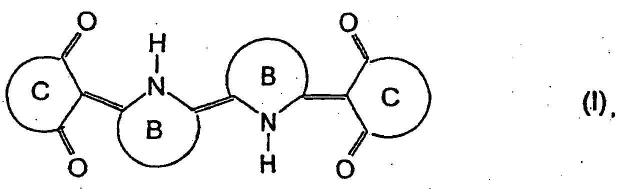 C étant un groupe alicyclique ou hétérocyclique avec une symétrie C₂ᵥ,
et B représente un ortho-arylène en C₆-C₁₈.

2. Composé selon la revendication 1, **caractérisé en ce que** B représente un ortho-phénylène ou 2,3-naphtylène.

3. Composé selon la revendication 1 ou 2, **caractérisé en ce que** le cycle C correspond à un système cyclique de formules (a) à (d) 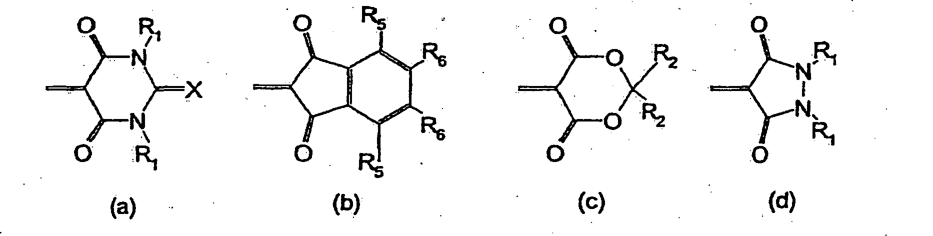 R₁ et R₂, indépendamment l'un de l'autre, représentent un atome d' hydrogène, un groupe alkyle en C₁-C₂₅, cycloalkyle en C₅-C₁₂, aryle en C₆-C₂₄, (alkyle en Ci-C₂₅)-(aryle en C₆-C₁₀), un reste hétéroaromatique présentant 1, 2 ou 3 hétéroatomes pris dans le groupe des atomes de N, O et S, -(CH₂)ₙ-COR₃ ou -(CH₂)ₘ-OR₄,
où
R³ représente un groupe hydroxy, alkoxy en C₁-C₂₅ non substitué ou substitué une ou plusieurs fois par un substituant hydroxy ou amino, (alkyl en C₁-C₂₅)amino, di (alkyl en C₁-C₂₅)amino, (alkyl en C₁-C₂₅)-(aryl en C₆₋C₂₄) amino, (aryl en C₆-C₂₄) amino, di(aryl en C₆₋C₂₄) amino ou (alcényl en C₂-C₂₄) oxy, et
R₄ représente un atome d'hydrogène ou un groupe -CO-(alkyle en C₁-C₂₅), et n et m, indépendamment l'un de l'autre, sont des entiers de 0 à 6, de préférence de 1 à 4, et où dans R₁, R₂, R₃ et R₄ une unité C-C peut aussi être remplacée par une unité éther C-O-C, et
X représente =O, =S ou =NR₂, et
R₅ et R₆, indépendamment l'un de l'autre, représentent un atome d'hydrogène, un atome d'halogène, un groupe CN, R₁, OR₁, SR₁, NR₁R₂, NO₂, SO₂(OR₁), SO₂R₁, SO₂NR₁R₂ ou PO₂(OR₁).

4. Composé selon une ou plusieurs des revendications 1 à 3, **caractérisé en ce que** R₁ et R₂. sont identiques ou différents et représentent un atome d'hydrogène, un groupe alkyle en C₁-C₁₈, benzyle, cycloalkyle en C₅-C₆, aryle en C₆-C₁₀, pyridyle, pyrryle, thiényle, imidazolyle, oxazolyle, thiazolyle, pyrimidyle, hydroxycarbonyl(alkyle en C₀₋C₁₈), (alkoxy en C₁-C₁₈) carbonyl-(alkyle en C₀-C₁₈), aminocarbonyl- (alkyle en C₀-C₁₈), (alkyl en C₁-C₁₈)-aminocarbonyl- (alkyle en C₀-C₁₈), (aryl en C₆-C₁₀) - aminocarbonyl-(alkyle en C₀-C₁₈), di (alkyl en C₁-C₁₈)-aminocarbonyl- (alkyle en C₀-C₁₈), (alkyl en C₁-C₁₈)-(aryl en C₆-C₁₀) aminocarbonyl- (alkyle en C₀-C₁₈) ou di (aryl en C₆-C₁₀) aminocarbonyl- (alkyle en C₀-C₁₈).

5. Composé selon une ou plusieurs des revendications 1 à 4, **caractérisé en ce que** R₅ et R₆ sont identiques ou différents et représentent un atome d' hydrogène, Cl, Br, un groupe alkyle en C₁-C₁₈, cycloalkyle en C₅-C₆, aryle en C₆-C₁₀, benzyle, pyridyle, pyrryle, thiényle, imidazolyle, oxazolyle, thiazolyle, pyrimidyle, alkoxy en C₁-C₁₈, (aryl en C₆₋C₁₀)oxy, (alkyle en C₁-C₁₈)thio, (aryl en C₆-C₁₀) thio, (alkyl en C₁-C₁₈)amino, (aryl en C₆-C₁₀) amino, di (alkyl en C₁-C₁₈)amino, (alkyl en C₁-C₁₈)-(aryle en C₆₋C₁₀)amino, di (aryl en C₆-C₁₀) amino, SO₃H, (alkoxy en C₁₋C₁₈)-sulfonyle, (alkyl en C₁-C₁₈)sulfonyle ou di (alkyl en C₁-C₁₈)aminosulfonyle.

6. Composé de formule (2) ou (3) selon la revendication 3, 4 ou 5,

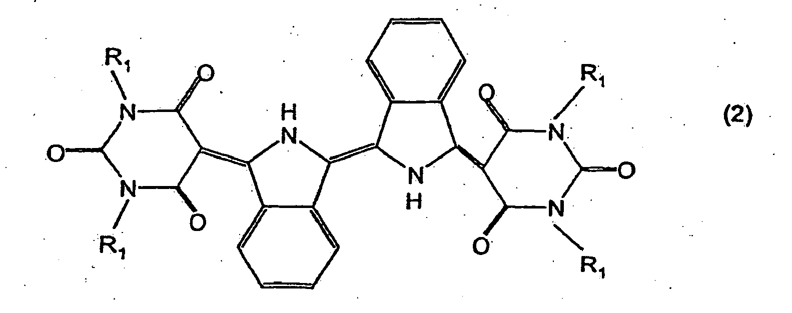

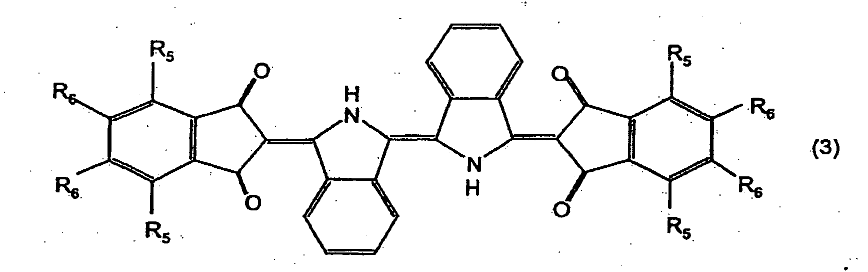

7. Procédé pour la préparation d'un composé selon une ou plusieurs revendications 1 à 6, **caractérisé en ce qu'**on condense un composé de formule (III), 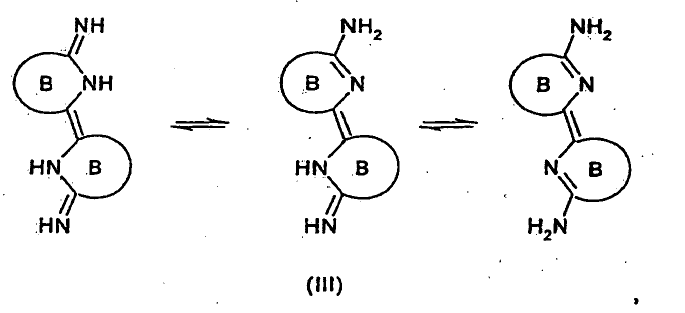 sur au moins 2 équivalents molaires d'un composé cyclique de formule (IV)

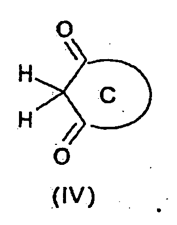

8. Utilisation d'un composé selon une ou plusieurs des revendications 1 à 6 pour la teinture ou la pigmentation de matières organiques ou inorganiques de haut ou de bas poids moléculaire, en particulier de matières organiques de haut poids moléculaire.

9. Utilisation selon la revendication 8 en tant que matière colorante dans les peintures à base d'eau ou d'huile, les vernis, les peintures de camouflage, pour la teinture dans la masse, pour la teinture dans la masse ou la pigmentation de matières synthétiques, dans les encres d'impression, dans la teinture de papier dans la masse, pour la semence, les vernis en poudre, pour la préparation d'encres, d'encres à jet d'encre à base d'eau ou d'huile, d'encres en microémulsion et d'encres qui opèrent selon le principe hot-melt.

10. Utilisation selon la revendication 8 en tant que matière colorante dans les toners électrophotographiques et dévéloppateurs, pour filtres colorés, pour encres électroniques, ainsi que dans les couches optiques pour le stockage optique de données.

11. Composition renfermant une matière organique ou inorganique de haut poids moléculaire ou de bas poids moléculaire, et un composé selon une ou plusieurs des revendications 1 à 6 en une quantité de 0,005 à 70 % en masse par rapport à la matière organique ou inorganique.
